# NEUE EUROPÄISCHE PATENTSCHRIFT

(11) **EP 1 040 815 B2**
(45) Veröffentlichungstag und Bekanntmachung des Hinweises auf die Entscheidung über den Einspruch: **22.06.2011**
(45) Hinweis auf die Patenterteilung: 24.08.2005
(21) Anmeldenummer: 00106766.9
(22) Anmeldetag: 29.03.2000
(51) Int. Cl.: A61K 8/44, A61K 8/34, A61Q 7/00

(54) **Haartonikum mit L-Arginin als Wirkstoff**
Hair tonic comprising L-Arginine as active compound
Lotion capillaire avec l'arginine comme principe actif

(30) Priorität: 01.04.1999 DE 29906038 U
(43) Veröffentlichungstag der Anmeldung: 04.10.2000
(73) Patentinhaber: Maindok, Friedrich, 58313 Herdecke (DE)
(72) Erfinder: Maindok, Friedrich, 58313 Herdecke (DE)
(74) Vertreter: Gesthuysen, von Rohr & Eggert

(56) Entgegenhaltungen:
- WO-A-94/09750
- JP-A- 05 286 834
- US-A- 3 778 502
- COSMETICS AND TOILETRIES Bd. 93, 1978, Seiten 49 - 50
- J. PHARM. PHARMACOL. Bd. 42, 1990, Seiten 71 - 72
- J. OF PHARMACEUTICAL SCIENCES Bd. 83, Nr. 10, 1994, Seiten 1508 - 1510
- J. PHARM. SCIENCES, [Online] Bd. 84, Nr. 6, 1995, Seiten 688 - 691 Gefunden im Internet: <URL:http://www3.interscience.wiley.com/cgi -bin/abstract/113295797/ABSTRACT> [gefunden am 2007-10-09]

## Beschreibung

Die Erfindung betrifft ein Haarwasser oder ein Haartonikum mit dem Zweck Haarausfall zu verhindern und den Haarwuchs zu fördern.

Haarwuchsmittel wie Thioctate einschließlich Argininthioctat sind bereits im Derwent-Ref.: 87-25 38 62/36 beschrieben. Die DE-PS-31 18 882 nennt verschiedene Gründe für Haarausfall und erwähnt weiterhin, daß bisher verschiedene Mittel gegen Haarausfall erprobt wurden. Die im darin zitierten Stand der Technik erzielten Erfolge hatten jedoch häufig gesundheitliche Schädigungen zur Folge, weshalb trotz der schwierigen Aufgabe und zahlreicher Fehlversuche die Suche nach geeigneten Mitteln nicht endet. Weil das Fehlen einer einzigen Aminosäure angeblich den Keratinaufbau unmöglich mache, beschreibt diese Patentschrift Haarwasser zum Einmassieren mit verschiedenen Wirkstoffen, umfassend 15 Aminosäuren und Knoblauchextrakt, wobei das Verhältnis von Histidin : Lysin : Arginin 1 : 4 : 12 beträgt.

Die FR-PS-2 609 393 nennt Aminosäuren einschließlich Arginin als kosmetische und pharmazeutische Wirkstoffe zur Epidermisbehandlung, schlägt aber gegen Haarausfall eine Lotion vor, die Plasmahydrolysat, Keratinhydrolysat, Fruchtwasser, Glycerin und Methylparaben enthält.

Die gegenseitige Beeinflussung der Wirkstoffe bei Anwendung dieser Mittel dürfte weitgehend unbekannt sein. Schädigende Nebenwirkungen sind oft erst spät zu erkennen, weshalb diese Mittel mangels bekannter Gegenanzeigen nicht unbedenklich sind. Darüber hinaus sind die Wirkstoffe teuer, teilweise von Verfall bedroht und die Wirkungen der Zerfalls- und Nebenprodukte weitgehend unbekannt.

Aus der DE 42 05 931 A1 ist die Verwendung einer einmolaren Elektrolytlösung enthaltend 1-mmol/l Argininhydrochlorid als Infusionslösung zur Behandlung von Haarausfall und zur Haarwuchsförderung bekannt, die im Volumenverhälnis von 2/8 mit Kochsalzlösung vermischt in das Gesäß oder in die Kopfhaut injiziert wird. Das Injizieren ist jedoch unangenehm bzw. lästig.

Aus der WO94/09750 sind haarwuchsfördernde Mittel, die L-Arginin enthalten, bekannt. Es wird jedoch in diesem Dokument keine Zusammensetzung offenbart, die sowohl Ethanol, Propylenglykol, L-Arginin und Wasser enthält.

Aufgabe der vorliegenden Erfindung ist es, ein wirksames Haartonikum bereitzustellen, mit dem die Gesundheitsrisiken und die Herstellungskosten möglichst gering gehalten werden.
Zur Lösung der erfindungsgemäßen Aufgabe wird ein Haartonikum aus L-Arginin und Lösungsmittel gemäß Anspruch 1 bereitgestellt; weitere, vorteilhafte Ausgestaltungen sind Gegenstand der Unteransprüche.
Erfindungsgemäß lassen sich selbst deaktivierte Haarwurzeln aktivieren, so daß sie wieder Haare bilden.
Das erfindungsgemäße Haartonikum ist eine stabile klare Lösung. Eine Änderung infolge unsachgemäßer Behandlung würde sofort auffallen.
Vorteilhafterweise fördern dermatologisch harmlose Lösungsmittel die Aufnahme des Wirkstoffs Arginin durch die Kopfhaut.
Duftstoffe können dem Haartonikum einen angenehmen Geruch geben.

### Beispiel 1

Ein weiteres erfindungsgemäßes Haartonikum ist eine Mischung folgender Bestandteile:

| | |
|---|---|
| 420 ml | Ethanol |
| 359 ml | Wasser |
| 200 ml | Propylenglykol |
| 20 g | L-Arginin |
| 1 ml | Parfüm |

Der Begriff Wasser umfaßt insbesondere Quellwasser, Trinkwasser, destilliertes Wasser, demineralisiertes Wasser und sterilisiertes Wasser.
Der Begriff Ethanol schließt handelsüblichen Alkohol, insbesondere vergällten Alkohol, ein.
Derartige Inhaltsstoffe sind im ersten Verzeichnis der in der Europäischen Union eingesetzten Inhaltsstoffe von Körperpflegemitteln veröffentlicht (Amtsblatt der Europäischen Gemeinschaften vom 1. Juni 1996). Dort wird vergällter Ethanol als Alcohol denat, Wasser als Aqua bezeichnet.

## Patentansprüche

1. Haartonikum, pro liter bestehend aus :
| | |
|---|---|
| 300 bis 800 ml | Ethanol |
| 50 bis 400 ml | Propylenglykol |
| 2 bis 300 g | L-Arginin |
| 50 bis 600 g | Wasser |
| 0 bis 20 g | Parfüm |

2. Haartonikum nach Anspruch 1, **gekennzeichnet durch** einen Gehalt an Propylenglykol von 150 bis 250 ml/l.

3. Haartonikum nach Anspruch 1 oder 2, **gekennzeichnet durch** einen Gehalt an L-Arginin von 10 bis 100 g/l.

4. Haartonikum nach einem der Ansprüche 1 bis 3, **gekennzeichnet durch** einen Wasseranteil von 150 bis 400 g/l.

5. Haartonikum, nach einem der Ansprüche 1 bis 4, **gekennzeichnet durch** einen Ethanol-Gehalt von 500 bis 700 ml/l.

## Claims

1. Hair tonic consisting of per liter:
| | |
|---|---|
| 300 to 800 ml | ethanol |
| 50 to 400 ml | propylene glycol |
| 2 to 300 g | L-arginine |
| 50 to 600 g | water |
| 0 to 20 g | perfume |

2. Hair tonic according to claim 1 **characterized by** a content of propylene glycol of 150 to 250 ml/l.

3. Hair tonic according to claim 1 or 2 **characterized by** a content of L-arginine of 10 to 100 g/l.

4. Hair tonic according to any of claims 1 to 3 **characterized by** a water content of 150 to 400 g/l.

5. Hair tonic according to any of claims 1 to 4 **characterized by** an ethanol content of 500 to 700 ml/l.

## Revendications

1. Tonique (lotion) capillaire consistant en par litre:
| | |
|---|---|
| 300 à 800 ml | de l'éthanol |
| 50 à 400 ml | du propylèneglycol |
| 2 à 300 g | de la L-arginine |
| 50 à 600 g | d'eau |
| 0 à 20 g | de parfum. |

2. Tonique capillaire selon la revendication 1 charactérisé par une teneur en propylèneglycol de 150 à 250 ml/l.

3. Tonique capillaire selon la revendication 1 ou 2 charactérisé par une teneur en L-arginine de 10 à 100 g/l.

4. Tonique capillaire selon l'une des revendications 1 à 3 charactérisé par une teneur en eau de 150 à 400 g/l.

5. Tonique capillaire selon l'une des revendication 1 à 4 charactérisé par une teneur en éthanol de 500 à 700 ml/l.
